Europäisches Patentamt

European Patent Office

Office européen des brevets

(11) Veröffentlichungsnummer: **0 513 508 A2**

# EUROPÄISCHE PATENTANMELDUNG

(21) Anmeldenummer: **92105521.6**

(22) Anmeldetag: **31.03.92**

(51) Int. Cl.⁵: **A61H 23/02**

(30) Priorität: **18.05.91 DE 4116358**

(43) Veröffentlichungstag der Anmeldung:
**19.11.92 Patentblatt 92/47**

(84) Benannte Vertragsstaaten:
**AT CH DE FR GB IT LI NL PT SE**

(71) Anmelder: **Rheinmagnet Horst Baermann GmbH**
**Ohlenhohnstrasse 23**
**W-5206 Neunkirchen-S. 1(DE)**

(72) Erfinder: **Baermann, Horst**
**Branderhof 9**
**W-5060 Bergisch Gladbach 1(DE)**

(74) Vertreter: **Stachow, Ernst-Walther et al**
**Lippert, Stachow, Schmidt & Partner,**
**Patentanwälte, Frankenforster Strasse**
**135-137, P.O. Box 30 02 08**
**W-5060 Bergisch Gladbach 1(DE)**

(54) **Verfahren und Vorrichtung zur Erzeugung einer Massagewirkung.**

(57) Bei einem Verfahren zur Erzeugung einer Massagewirkung auf Körperoberflächen, das von einer elektromagnetischen Einrichtung Gebrauch macht, werden zur Vermeidung von Bewegungseinschränkungen und Erzielung einer optimalen Massagewirkung ein oder mehrere Dauermagnete an einer zu behandelnden Körperoberfläche so angeordnet, daß Schwingungen, zu denen der bzw. die Magnete angeregt werden, auf die Körperoberfläche übertragen werden, und der bzw. die Magnete mit dem betreffenden Körperteil in ein magnetisches Wechsel- oder gepulstes Feld gebracht.
Eine Vorrichtung zur Durchführung eines solchen Verfahrens weist eine mit Wechsel- oder pulsierenden Gleichstrom beaufschlagte Spule (1), deren Innenabmessungen sich nach den Maßen der in diese eintauchenden Körperteile richtet, und eine oder mehrere an den Körperteilen zu befestigende Dauermagnete, z.B. einen ringförmigen Magneten (3), auf.

EP 0 513 508 A2

Die Erfindung betrifft ein Verfahren und eine Vorrichtung zur Erzeugung einer Massagewirkung auf Körperoberflächen, wobei von einer elektromagnetischen Einrichtung Gebrauch gemacht wird.

Bekannte Verfahren und Vorrichtungen zur Erzeugung einer Massagewirkung der obengenannten Art verwenden elektromotorisch oder durch einen Schwinganker angetriebene Vibratoren, die mit der zu behandelnden Körperoberfläche in Berührung gebracht werden. Der Vibrationsteil dieser Vorrichtungen steht dabei mit dem Antrieb in mechanischer Verbindung. Wird das Vibrationsteil zur Ausübung einer Massagewirkung mit dem zu behandelnden Körperteil in Berührung gebracht, so entsteht ebenfalls eine mechanische Kopplung zwischen dem betreffenden Körperteil und dem Antrieb der Vibratoren. Daher sind den Bewegungsmöglichkeiten des Vibrationsteils und der auf das betreffende Körperteil ausgeübten Massagewirkung Grenzen gesetzt.

Der Erfindung liegt die Aufgabe zugrunde, ein Verfahren und eine Vorrichtung zur Erzeugung einer Massagewirkung der eingangs genannten Art zu schaffen, wobei unter Vermeidung derartiger Beschränkungen eine optimale Massagewirkung auf die jeweilige Körperoberfläche ausgeübt werden kann.

Die Aufgabe wird erfindungsgemäß dadurch gelöst, daß bei einem Verfahren der genannten Art ein oder mehrere Dauermagnete an einer zu behandelnden Körperoberfläche so angeordnet werden, daß Schwingungen, zu denen der bwz. die Magnete angeregt werden, auf die Körperoberfläche übertragen werden, und der bzw. die Magnete mit dem betreffenden Körperteil in ein magnetisches Wechsel- oder gepulstes Feld gebracht werden.

In einem solchen Feld kann sich das zu behandelnde Körperteil mit dem bzw. den darauf angeordneten Magneten ohne die bisherige mechanische Kopplung mit dem Antrieb bewegen. Durch geeignete Magnetisierung des bzw. der Dauermagnete können diese in dem Feld zu beliebigen Schwingungen angeregt werden. Aufgrund der Art und Ausführungen der Schwingungen kann daher eine optimale Massagewirkung auf die zu behandelnde Körperoberfläche ausgeübt werden.

Eine erfindungsgemäße Vorrichtung zur Durchführung des obigen Verfahrens umfaßt einen oder mehrere Dauermagnete und eine mit Wechsel- oder pulsierendem Gleichstrom beaufschlagte Spule, deren Innenabmessungen so gewählt sind, daß das zu behandelnde Körperteil mit dem bzw. den darauf angeordneten Dauermagneten in die Spule eintauchbar und darin frei schwingbar sind.

Die Spule kann dabei in jeder geeigneten Form ausgebildet sein und eine oder mehrere Windungen einschließen. Sie kann weiterhin mit beliebigen Frequenzen beaufschlagt werden, die zweckmäßigerweise bis zu 200 Hz betragen.

Bevorzugt wird die Spule mit Frequenzen zwischen 1 Hz und 60 Hz beaufschlagt.

Bei einem besonderen Ausführungsbeispiel der Erfindung weisen der bzw. die Dauermagnete einen bzw. mehrere einstückige, ringförmige Magnete auf. Die ringförmigen Magnete können so bemessen sein, daß sie z.B. um einen Finger, ein Handgelenk oder Gliedmaßen angeordnet werden können. Die Innenabmessungen der Spule richten sich dann nach den Maßen der in diese eintauchenden und mit einem oder mehreren Ringen versehenen Körperteile.

Der oder die ringförmigen Magnete können in geeigneter Weise magnetisiert sein, um bestimmte Bewegungsabläufe hervorzurufen.

In einem bevorzugten Ausführungsbeispiel weist der ringförmige Magnet eine axiale Magnetisierung mit einem an einer Stirnseite angeordneten Pol und einem dazu entgegengesetzten, an der anderen Stirnseite angeordneten Pol auf. Wird z.B der Finger oder das Handgelenk mit einem derart magnetisierten Ringmagneten axial in die Spule eingetaucht, so ergibt sich beim Einschalten des Feldes eine axiale Hin- und Herbewegung des Ringmagneten, die eine entsprechende Massagewirkung auf den Finger bzw. das Handgelenk ausübt.

In einem anderen bevorzugten Ausführungsbeispiel weist der ringförmige Magnet eine zwei- oder mehrpolige axiale Magnetisierung mit zwei oder mehreren an einer Stirnseite angeordneten Polen und einer entsprechenden Anzahl dazu entgegengesetzter, an der anderen Stirnseite angeordneter Pole auf. Aufgrund der mehrpoligen axialen Magnetisierung führt der Ring im magnetischen Wechsel- oder gepulsten Feld eine taumelnde Bewegung aus.

In einem weiteren bevorzugten Ausführungsbeispiel weist der ringförmige Magnet eine diametrale Magnetisierung mit einem in einer Ringhälfte angeordneten Pol und einem dazu entgegengesetzten, in der anderen Ringhälfte angeordneten Pol auf. Diese Magnetisierung ruft im Magnetfeld der Spule eine Kippbewegung des Magnetringes hervor.

Darüber hinaus können alle anderen geeigneten Magnetisierungsarten gewählt werden.

In einer anderen Ausführung kann der Dauermagnet als kugelförmiger Magnet ausgebildet sein, so daß er von einer Hand umschlossen werden kann. In diesem Fall können je nach Magnetisierung des kugelförmigen Magneten bestimmte Vibrationsbewegungen auf die Hand ausgeübt werden.

Der Dauermagnet kann auch beliebig andere Gestaltungen annehmen. Er kann z.B. flächig oder gekrümmt oder als Manschette ausgebildet sein. Insbesondere kann er auch anatomisch dem zu behandelnden Körperteil angepaßt sein.

Darüber hinaus kann der Dauermagnet an seiner Oberfläche Noppen oder dergleichen zur Verstärkung der Massagewirkung aufweisen.

Andererseits kann der Dauermagnet in einen entsprechend dem zu behandelnden Körperteil anatomisch geformten Kunststoffkörper eingebettet sein.

Der Dauermagnet kann einzeln oder in Kombination mit mehreren dieser Magnete in Bandagen aller Art oder in Handschuhen und dergleichen durch Einweben, Kleben sowie durch andere bekannte Verfahren eingearbeitet sein.

Er kann andererseits auch selbstklebend oder mit Hilfe eines Kebeträgers auf der zu behandelnden Körperoberfläche befestigt sein.

Der bzw. die Dauermagnete bestehen vorzugsweise aus hochkoerzitiven Werkstoffen, wie Barium- oder Strontiumferriten oder Seltenerdwerkstoffen, wie NdFeB, oder Kombinationen dieser Werkstoffe. Die Werkstoffe können in gesinterter Form vorliegen oder aus pulverförmigem Material bestehen, das mit feiner und möglichst homogener Verteilung in einem duro- oder thermoplastischen Kunststoffmaterial eingebettet ist und z.B. durch Spritzen oder Verpressen in die gewünschte Form gebracht wird.

Im folgenden werden einige Ausführungsbeispiele der Erfindung anhand der Zeichnung näher beschrieben. Es zeigen:

Fig. 1    einen Querschnitt durch eine Spule mit einem darin angeordneten ringförmigen Magneten,

Fig. 2    eine andere Ausführung des ringförmigen Magneten und

Fig. 3    eine weitere Ausführung des ringförmigen Magneten.

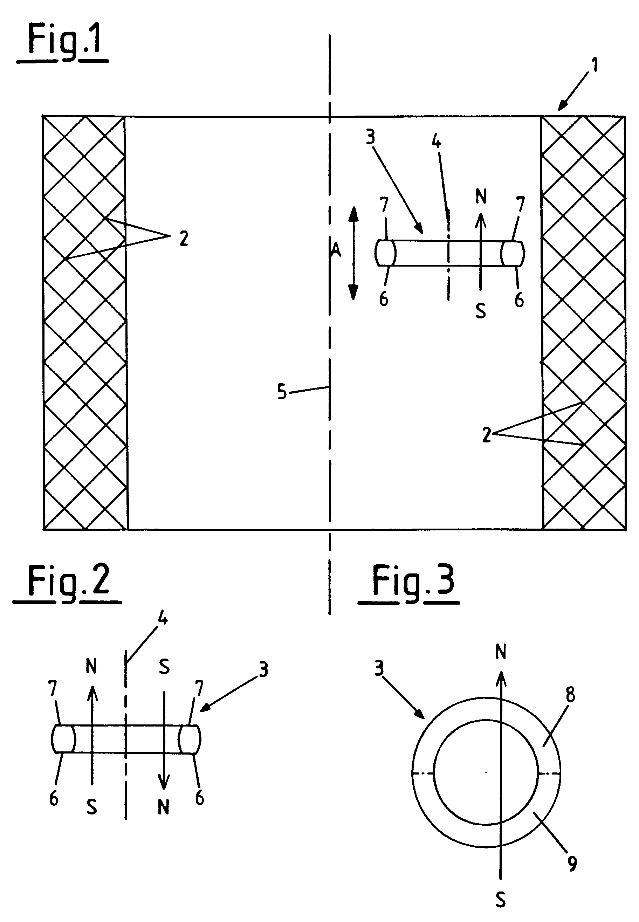

Das in Fig. 1 dargestellte Ausführungsbeispiel besteht aus einer Spule 1 mit einer Vielzahl von Windungen 2 und einem ringförmigen Magneten 3, der an einem (in der Zeichnung nicht dargestellten) Finger so in die Spule 1 eintaucht, daß die Achse 4 des ringförmigen Magneten 3 parallel zur Achse 5 der Spule 1 angeordnet ist.

Der ringförmige Magnet 3 ist axial magnetisiert mit einem an einer Stirnseite 6 angeordneten Pol S und einem dazu entgegengesetzten, an der anderen Stirnseite 7 angeordneten Pol N.

Wird die Spule 1 mit Wechsel- oder einem pulsierenden Gleichstrom beaufschlagt, so bewegt sich der ringförmige Magnet 3 mit der Frequenz des in der Spule 1 erzeugten Magnetfeldes in axialer Richtung hin und her. Mit dieser Frequenz wird eine Massagewirkung auf den Finger ausgeübt. Die dabei auftretenden axialen Kräfte F ergeben sich in Abhängigkeit vom Magnetfeld H nach der folgenden Beziehung:

$$F = c \times H^2.$$

Durch Wahl anderer Magnetisierungsarten für den ringförmigen Magneten 3 können andere Bewegungsabläufe hervorgerufen werden.

Fig. 2 zeigt einen axial zwei-polig magnetisierten ringförmigen Magneten mit zwei an einer Stirnseite 6 angeordneten Polen S und N und zwei dazu entgegengesetzten, an der anderen Stirnseite 7 angeordneten Polen N bzw. S.

Wird dieser ringförmige Magnet in das von der Spule 1 erzeugte Magnetfeld gebracht, so wird ein taumelnder Bewegungsablauf hervorgerufen. Diese Bewegung wird auf den Finger übertragen und übt eine entsprechende Massagewirkung auf den Finger aus.

Bei dem in Fig. 3 dargestellten dritten Ausführungsbeispiel ist der ringförmige Magnet 3 diametral magnetisiert mit einem in einer Ringhälfte 8 angeordneten Pol N und einem in der anderen Ringhälfte 9 angeordneten Pol S.

Wird dieser ringförmige Magnet 3 in die Spule 1 eingetaucht, so daß die Achse 4 des Magneten parallel zur Achse 5 der Spule 1 steht, dann führt der Magnet 3 mit der Frequenz des in der Spule 1 erzeugten Magnetfeldes eine alternierende Kippbewegung durch. Auch diese Kippbewegung übt eine bestimmte Massagewirkung auf den im ringförmigen Magneten 3 steckenden Finger aus.

Die in der Zeichnung dargestellten ringförmigen Magnete 3 bestehen aus hochkoerzitiven Werkstoffen, wie z.B. Barium- oder Strontiumferrit oder Seltenerdmagnetwerkstoffen, wie z.B. NdFeB oder Kombinationen dieser Werkstoffe. Sie können in gesinterter Ausführung vorliegen oder aus pulverförmigem Material bestehen, das in feiner oder möglichst homogener Verteilung in einem duro- oder thermoplastischem Material eingebettet ist und z.B. durch Spritzen oder Verpressen in die gewünschte Form gebracht ist.

Das Prinzip des oben beschriebenen Massageverfahrens kann ebenso durch anders gestaltete Dauermagneten, die in geeigneter Weise an den zu behandelnden Körperoberflächen angeordnet werden, verwirklicht werden.

## Bezugszeichenliste

| N | Nordpol |
|---|---------|
| S | Südpol |
| 1 | Spule |
| 2 | Windung |
| 3 | ringförmiger Magnet |
| 4 | Achse des Magneten |
| 5 | Achse der Spule |
| 6 | Stirnseite |

7  Stirnseite
8  Ringhälfte
9  Ringhälfte

## Patentansprüche

1. Verfahren zur Erzeugung einer Massagewirkung auf Körperoberflächen, das von einer elektromagnetischen Einrichtung Gebrauch macht, **dadurch gekennzeichnet**, daß ein oder mehrere Dauermagnete an einer zu behandelnden Körperoberfläche so angeordnet werden, daß Schwingungen, zu denen der bzw. die Magnete angeregt werden, auf die Körperoberfläche übertragen werden, und der bzw. die Magnete mit dem betreffenden Körperteil in ein magnetisches Wechsel- oder gepulstes Feld gebracht werden.

2. Vorrichtung zur Durchführung des Verfahrens nach Anspruch 1, **gekennzeichnet durch** einen oder mehrere Dauermagnete und eine mit Wechsel- oder pulsierendem Gleichstrom beaufschlagte Spule (1), deren Innenabmessungen so gewählt sind, daß das zu behandelnde Körperteil mit dem bzw. den darauf angeordneten Dauermagneten in die Spule (1) eintauchbar und darin frei schwingbar ist.

3. Vorrichtung nach Anspruch 2, **dadurch gekennzeichnet**, daß die Spule (1) mit Frequenzen zwischen 1 Hz und 60 Hz beaufschlagt ist.

4. Vorrichtung nach Anspruch 2 oder 3, **dadurch gekennzeichnet**, daß der bzw. die Dauermagnete einen bzw. mehrere einstückige, ringförmige Magnete (3) aufweisen.

5. Vorrichtung nach Anspruch 4, **dadurch gekennzeichnet**, daß der ringförmige Magnet (3) eine axiale Magnetisierung mit einem an einer Stirnseite (6) angeordneten Pol (S) und einem dazu entgegengesetzten, an der anderen Stirnseite (7) angeordneten Pol (N) aufweist.

6. Vorrichtung nach Anspruch 4, **dadurch gekennzeichnet**, daß der ringförmige Magnet (3) eine zwei- oder mehrpolige axiale Magnetisierung mit zwei oder mehreren an einer Stirnseite (6) angeordneten Polen (S, N) und einer entsprechenden Anzahl dazu entgegengesetzter, an der anderen Stirnseite (7) angeordneter Pole (N, S) aufweist.

7. Vorrichtung nach Anspruch 4, **dadurch gekennzeichnet**, daß der ringförmige Magnet (3) eine diametrale Magnetisierung mit einem in einer Ringhälfte (8) angeordneten Pol (N) und einem dazu entgegengesetzten, in der anderen Ringhälfte (9) angeordneten Pol (S) aufweist.

8. Vorrichtung nach Anspruch 2 oder 3, **dadurch gekennzeichnet**, daß der Dauermagnet als kugelförmiger Magnet ausgebildet ist, so daß er von einer Hand umfaßt werden kann.

9. Vorrichtung nach Anspruch 2 oder 3, **dadurch gekennzeichnet**, daß der Dauermagnet anatomisch dem zu behandelnden Körperteil angepaßt ist.

10. Vorrichtung nach Anspruch 2 oder 3, **dadurch gekennzeichnet**, daß der Dauermagnet an seiner Oberfläche Noppen aufweist.

11. Vorrichtung nach Anspruch 2 oder 3, **dadurch gekennzeichnet**, daß der Dauermagnet in einen entsprechend dem zu behandelnden Körperteil anatomisch geformten Kunststoffkörper eingebettet ist.

12. Vorrichtung nach Anspruch 2 oder 3, **dadurch gekennzeichnet**, daß der Dauermagnet einzeln oder in Kombination mit mehreren Magneten in Bandagen aller Art oder in Handschuhen oder dergleichen durch Einweben, Kleben sowie durch andere bekannte Verfahren eingearbeitet ist.

13. Vorrichtung nach Anspruch 2 oder 3, **dadurch gekennzeichnet**, daß der Dauermagnet einzeln oder in Kombination mit mehreren Magneten selbstklebend oder mit Hilfe eines Klebeträgers auf der zu behandelnden Körperoberfläche befestigbar ist.

14. Vorrichtung nach einem der Ansprüche 2 bis 13, **dadurch gekennzeichnet**, daß der bzw. die Dauermagnete aus hochkoerzitiven Werkstoffen, wie Barium- oder Strontiumferriten oder Seltenerdwerkstoffen, wie z.B. NdFeB, oder Kombinationen dieser Werkstoffe bestehen.

15. Vorrichtung nach Anspruch 14, **dadurch gekennzeichnet**, daß die Werkstoffe in gesinterter Form vorliegen.

16. Vorrichtung nach Anspruch 14, **dadurch gekennzeichnet**, daß der bzw. die Dauermagnete aus pulverförmigem Material bestehen, das in feiner und möglichst homogener Verteilung in einem duro- oder thermoplastischem Kunststoffmaterial eingebettet und z.B. durch Spritzen oder Verpressen in die gewünschte Form gebracht ist.

## Fig.1

## Fig.2

## Fig.3